# EUROPÄISCHE PATENTANMELDUNG

(11) **EP 4 821 517 A1**
(43) Veröffentlichungstag der Anmeldung: **14.10.2026**
(21) Anmeldenummer: 26170999.2
(22) Anmeldetag: 07.04.2026
(51) Int. Cl.: A61M 1/16, A61M 1/36

(54) **EXTRAKORPORALE BLUTBEHANDLUNGSMASCHINE UND VERFAHREN ZUM PRIMEN EINES DIALYSATORS**

(30) Priorität: 09.04.2025 DE 102025114056
(71) Anmelder: B. Braun Avitum AG, 34212 Melsungen (DE)
(72) Erfinder: WUERSCHMIDT, Tobias, 34346 Hann. Münden (DE)
(74) Vertreter: Winter, Brandl - Partnerschaft mbB

(57) **Zusammenfassung**

Die Offenbarung betrifft eine extrakorporale Blutbehandlungsmaschine mit einem Dialysator (4) mit einer Blutseite und einer Dialysierflüssigkeitsseite, einem Dialysierflüssigkeitskreislauf mit einem Dialysierflüssigkeitszulauf (12), welcher ein Zulaufventil (14) aufweist, einem Dialysierflüssigkeitsablauf (18), welcher ein Ablaufventil (20) aufweist, wobei der Dialysierflüssigkeitskreislauf über den Dialysierflüssigkeitszulauf (12) sowie den Dialysierflüssigkeitsablauf (18) mit der Dialysierflüssigkeitsseite des Dialysators (4) verbunden ist. Dabei weist der Dialysierflüssigkeitskreislauf einen ersten Zusatz-Strömungspfad (26) mit einem Zusatzventil (24) in Form eines 3/2-Wegeventils und einen zweiten Zusatz-Strömungspfad (28) mit einem Rückschlagventil (30) auf, über welche der Dialysierflüssigkeitszulauf (12) und der Dialysierflüssigkeitsablauf (18) des Dialysierflüssigkeitskreislaufs fluidisch verbindbar sind. Ferner weist die Blutbehandlungsmaschine eine Steuereinheit (38) auf, die eingerichtet ist, das Zusatzventil (24), das Zulaufventil (14) sowie das Ablaufventil (20) beim Primen derart zu steuern, dass eine Flussrichtung auf der Dialysierflüssigkeitsseite des Dialysators (4) beim Primen im Vergleich zu einer Flussrichtung bei einer Therapie umkehrbar ist.

## Beschreibung

### Technisches Gebiet

Die vorliegende Offenbarung betrifft eine extrakorporale Blutbehandlungsmaschine, insbesondere eine Dialysemaschine, mit einem Hohlfasern aufweisenden Dialysator. Außerdem betrifft die Offenbarung ein Verfahren zum automatischen Primen eines Dialysators der extrakorporalen Blutbehandlungsmaschine.

### Hintergrund der Offenbarung

Auf dem Gebiet der extrakorporalen Blutbehandlung, insbesondere der Dialyse, ist es üblich, dass Patienten, deren Nieren nicht mehr in der Lage sind, Abfallprodukte aus dem Blut zu filtern, regelmäßig behandelt werden. Diese Behandlungen erfordern den Einsatz von Dialysemaschinen, die das Blut des Patienten außerhalb des Körpers (extrakorporal) reinigen, indem sie es durch einen Dialysator leiten, in dem die Reinigung über eine semipermeable Membran stattfindet. Bekannte Systeme weisen typischerweise einen Hohlfaserdialysator auf, der eine Blutseite und eine Dialysierflüssigkeitsseite aufweist, die durch eine semipermeable Membran getrennt sind. Das Prinzip der Dialyse basiert auf dem Diffusionsprozess, bei dem Abfallstoffe und überschüssige Flüssigkeiten durch die Membran in eine Dialysierflüssigkeit (Dialysat) übergehen, während das gereinigte Blut zum Patienten zurückgeführt wird.

Ein wesentlicher Schritt vor Beginn jeder Dialyse ist das Priming bzw. Primen des Dialysators, d.h. das Vorbereiten und Füllen bzw. Spülen des Dialysators/ Filters mit einer speziellen Flüssigkeit, insbesondere einer Spülflüssigkeit/ Primingflüssigkeit, um Luftblasen aus dem Dialysator, insbesondere der Membran des Dialysators, zu entfernen. Luftblasen im Dialysator können zu ernsthaften Komplikationen führen, beispielsweise Luftembolien beim Patienten. Gemäß bekannter Verfahren wird das Priming bzw. Primen manuell oder halbautomatisch durchgeführt, indem Flüssigkeit durch den Dialysator und die Schlauchsysteme gepumpt wird oder durch einen manuellen Klopfvorgang durch den Bediener, um Luft zu verdrängen. Konventionelle Hohlfaserdialysatoren werden üblicherweise zunächst blutseitig gefüllt, bevor die Dialysierflüssigkeitsseite gefüllt wird. Dabei ist ein Drehen des Dialysators erforderlich, da während der Therapie die Flüssigkeitsströme auf beiden Seiten in übliche Weise gegenläufig verlaufen. Auf diese Weise können Schadstoffe, Abfallprodukte sowie überschüssiges Wasser am besten aus dem Blut entfernt und mit der Dialysierflüssigkeit abtransportiert werden. Beispielsweise beschreibt die EP 3 174 571 B1 eine gattungsgemäße extrakorporale Blutbehandlungsmaschine mit einem Hohlfaserdialysator.

Trotz der beträchtlichen Fortschritte auf dem Gebiet der Dialysetechnologie und der Automatisierung von Prozessen besteht nach wie vor ein Bedarf an verbesserten Methoden und möglichst kostengünstiges Vorrichtungen, die ein effizienteres, sichereres und vor allem ein automatisiertes Priming, d. h. ohne einen zusätzlichen Drehvorgang des Dialysators durch den Operator, ermöglichen. Insbesondere besteht die Herausforderung darin, Luftblasen effektiv und zuverlässig aus der semipermeablen Membran des Dialysators zu entfernen, um das Risiko von Luftembolien zu minimieren und die Sicherheit des Patienten zu gewährleisten.

Allerdings liegen bei solchen Lösungen immer noch Grenzen hinsichtlich der Automatisierung des Priming-Prozesses und der Effektivität bei der Entfernung von Luftblasen aus der semipermeablen Membran des Dialysators vor. Abgesehen davon, besteht die zusätzlich Herausforderung darin, diese automatisierten Priming-Prozesse effizient, kostengünstig und möglichst platzeinsparend umzusetzen.

### Kurzbeschreibung der vorliegenden Offenbarung

Daher liegt der vorliegenden Offenbarung die Aufgabe zugrunde, die vorstehend beschriebenen Nachteile zu vermeiden oder wenigstens abzumildern und insbesondere eine extrakorporale Blutbehandlungsmaschine bereitzustellen, welche eine möglichst vollständige Entfernung von Luft bzw. Luftblasen während eines Priming-Vorgangs effizient, kostengünstig, platzsparend und insbesondere automatisiert ermöglicht.

Diese Aufgabe wird durch eine extrakorporale Blutbehandlungsmaschine gemäß den Merkmalen des Anspruchs 1, durch ein Verfahren zum automatischen Primen eines Dialysators einer extrakorporalen Blutbehandlungsmaschine gemäß den Merkmalen des Anspruchs 9 und durch ein Computerprogramm gemäß den Merkmalen des Anspruchs 12 gelöst. Vorteilhafte Ausführungsformen sind in den Unteransprüchen beansprucht und/oder werden nachfolgend erläutert.

Die Offenbarung betrifft demzufolge zunächst eine extrakorporale Blutbehandlungsmaschine, insbesondere eine Dialysemaschine, welche einen Dialysator mit einer Blutseite/ Blutkammer und einer Dialysierflüssigkeitsseite/ Dialysierflüssigkeitskammer, die voneinander vorzugsweise durch eine semipermeable (halbdurchlässige) Membran getrennt sind, und vorzugsweise einen extrakorporalen Blutkreislauf aufweist, welcher über einen venösen Blutschlauchabschnitt und einen arteriellen Blutschlauchabschnitt mit der Blutseite des Dialysators verbunden ist. Ferner weist die Behandlungsmaschine einen Dialysierflüssigkeitskreislauf mit einem Dialysierflüssigkeitszulauf, welcher ein Zulaufventil aufweist, vorzugsweise in Form eines 2/2-Wegeventils, einen Dialysierflüssigkeitsablauf, welcher ein Ablaufventil aufweist, vorzugsweise in Form eines 2/2-Wegeventils, sowie vorzugsweise eine Ablaufpumpe in dem Dialysierflüssigkeitsablauf auf. Der Dialysierflüssigkeitskreislauf ist über den Dialysierflüssigkeitszulauf und den Dialysierflüssigkeitsablauf mit der Dialysierflüssigkeitsseite des Dialysators verbunden. Dabei weist der Dialysierflüssigkeitskreislauf einen ersten Zusatz-Strömungspfad mit einem Zusatzventil in Form eines 3/2-Wegeventils mit drei Anschlüssen und zwei Schaltstellungen und einen zweiten Zusatz-Strömungspfad mit einem Rückschlagventil auf, über die der Dialysierflüssigkeitszulauf und der Dialysierflüssigkeitsablauf des Dialysierflüssigkeitskreislaufs fluidisch verbindbar sind. Zudem weist die Blutbehandlungsmaschine eine Steuereinheit auf, die eingerichtet ist, das Zusatzventil, das Zulaufventil sowie das Ablaufventil beim Primen derart zu steuern, dass eine Flussrichtung auf der Dialysierflüssigkeitsseite des Dialysators beim Primen im Vergleich zu einer Flussrichtung bei einer Therapie umkehrbar ist.

Anders ausgedrückt bildet ein (Hohlfaser-)Dialysator das zentrale Element der extrakorporalen Blutbehandlungsmaschine. Dieser Dialysator ist in zwei Bereiche unterteilt, die Blutseite und die Dialysierflüssigkeitsseite. Beide Seiten sind vorzugsweise durch eine semipermeable Membran voneinander getrennt. Diese Anordnung ermöglicht den Austausch von Stoffen zwischen Blut und Dialysierflüssigkeit, beispielsweise Wasser von der Blutseite zur Dialysierflüssigkeitsseite, während gleichzeitig verhindert wird, dass Blutzellen und größere Moleküle die Membran passieren. Der Austausch zwischen diesen zwei Seiten erfolgt somit insbesondere durch den osmotischen Austausch über die semipermeable Membran.

Des Weiteren sind zwei Zusatz-Strömungspfade als fluidische Verbindungen des Dialysierflüssigkeitszulaufs und Dialysierflüssigkeitsablaufs auf der Seite des Dialysierflüssigkeitskreislaufs angeordnet. Hierbei ist einer der Zusatz-Strömungspfade mit einem 3/2-Wegeventil als Zusatzventil und der andere Zusatz-Strömungspfad mit einem passiven Rückschlagventil versehen.

Das 3/2-Wegeventil ist ein Ventil mit drei Anschlüssen und zwei Schaltstellungen, welches derart aufgebaut ist, einen Eingangsfluss über die zwei Schaltstellungen auf zwei verschiedene Weisen, beispielsweise in zwei verschiedene Wege aufzuteilen. Beispielsweise kann das 3/2-Wegeventil eingerichtet sein, in einer ersten Schaltstellung einen Eingangsfluss von einem ersten Anschluss zu einem zweiten Anschluss zu leiten, und in einer zweiten Schaltstellung den Eingangsfluss von dem ersten Anschluss zu einem dritten Anschluss zu leiten. Das 3/2-Wegeventil kann alternativ auch eingerichtet sein, in der ersten Schaltstellung den Eingangsfluss von dem ersten Anschluss zu dem zweiten Anschluss oder zu dem dritten Anschluss zu leiten, und in der zweiten Schaltstellung den Eingangsfluss zu sowohl dem zweiten Anschluss als auch zu dem dritten Anschluss zu leiten.

Das Rückschlagventil ist hingegen ausgebildet, den Flussweg des entsprechenden Zusatz-Strömungspfades nur in eine Richtung freizugeben. Das Rückschlagventil ist vorzugsweise eingerichtet, insbesondere während einer Therapie, wenn ein Druckunterschied zwischen dem zulaufenden und dem ablaufenden Fluss auf der Dialysierflüssigkeitsseite geringer als der Öffnungswiderstand des Rückschlagventils ist, den Flussweg des entsprechenden Zusatz-Strömungspfades zu sperren. Das Rückschlagventil ist weiter vorzugsweise eingerichtet, insbesondere während des Primens, also bei umgekehrter Flussrichtung, den Flussweg des entsprechenden Zusatz-Strömungspfades freizugeben. Somit kann durch ein Öffnen bzw. Schließen/ ein Aktivieren bzw. Deaktivieren des 3/2-Wegeventils des ersten Zusatz-Strömungspfads das Zulauf- bzw. Ablaufventil des Dialysierflüssigkeitszulaufs umgangen werden, wodurch die Flussrichtung der (Priming-) Flüssigkeit auf der Dialysierflüssigkeitsseite umkehrbar ist.

Dadurch kann vorzugsweise bewirkt werden, dass die (Priming-)Flüssigkeiten innerhalb und außerhalb der Hohlfasern, also auf beiden Seiten der semipermeablen Membran, während des Priming- bzw. Befüll- bzw. Spülvorgangs des Dialysators in gleicher Richtung strömen können. Besonders vorteilhaft ist es, wenn diese gleichgerichtete Flussrichtung der Flüssigkeiten entgegen der Schwerkraft erfolgt, da dies der natürlichen Fließrichtung der leichteren Luftblasen im Dialysator entspricht. Anders ausgedrückt entspricht eine derartige Flussrichtung der (Priming-)Flüssigkeit (auf der Blut- und der Dialysierflüssigkeitsseite) der Richtung, in welche die Luftblasen natürlich entweichen würden. Für eine nachfolgende Dialysetherapie kann das Zusatzventil in Form des 3/2-Wegeventils einfach entsprechend geschaltet, beispielsweise deaktiviert/ geschlossen, werden, um für die Behandlung vorteilhafte entgegengesetzte Flussrichtungen auf der Blutseite und der Dialysierflüssigkeitsseite automatisch einzustellen. Ein manuelles (Um-)Drehen des Dialysators ist demnach nicht notwendig. Vorzugsweise ist hierfür der arterielle Blutschlauchabschnitt des extrakorporalen Blutkreislaufs und der Dialysierflüssigkeitsablauf des Dialysierflüssigkeitskreislaufs auf einer, vorzugsweise dem Boden zugewandten, unteren Seite des Dialysators und entsprechend der venöse Blutschlauchabschnitt des extrakorporalen Blutkreislaufs und der Dialysierflüssigkeitszulauf des Dialysierflüssigkeitskreislaufs auf der anderen, oberen Seite des Dialysators angeordnet.

Der Einsatz/ Verbau eines einzigen 3/2-Wegeventils in einem Zusatz-Strömungspfad zur Ermöglichung der oben beschriebenen Flussumkehr kann bspw. den Einsatz von mehreren, insbesondere zwei, 2/2-Wegeventilen in den jeweiligen Zusatz-Strömungspfaden ersetzen, wodurch Kosten eingespart werden können. Ferner wird durch die Verringerung der Anzahl an (aktiven) Ventilen in den entsprechenden Zusatz-Strömungspfaden auf zusätzlich erforderliche Verschlauchung bzw. Verkabelung verzichtet, wodurch wiederum Materialkosten eingespart werden können. Des Weiteren wird hierdurch der erforderliche Bauraum der Blutbehandlungsmaschine reduziert.

In einem weiteren vorteilhaften Aspekt der Offenbarung kann der Dialysierflüssigkeitskreislauf einen Bypass-Pfad mit einem Bypass-Ventil aufweisen, über welches ein Bypass-Zustand der Blutbehandlungsmaschine einstellbar ist. Bei geöffnetem Bypass-Ventil wird die Dialysierflüssigkeit also am Dialysator vorbeigeführt, und zwar insbesondere ohne das Zulaufventil bzw. Ablaufventil des Dialysierflüssigkeitskreislaufs zu passieren.

In einer weiteren bevorzugten Ausführungsform der Offenbarung kann das Zusatzventil im Bypass-Pfad stromaufwärts des Bypass-Ventils bzw. in Flussrichtung vor diesem angeordnet sein und die Steuereinheit kann eingerichtet und vorbereitet sein, die Flussrichtung auf der Dialysierflüssigkeitsseite des Dialysators durch ein Aktivieren bzw. Öffnen des Zusatzventils, was gemäß dem bevorzugten Ausführungsbeispiel damit einhergeht, dass der Eingangsfluss von dem ersten Anschluss zu sowohl dem zweiten Anschluss als auch zu dem dritten Anschluss geleitet wird, ein Schließen des Bypass-Ventils, und ein Schließen des Zulaufventils sowie des Ablaufventils umzukehren.

Anders ausgedrückt kann das Zusatzventil in Form des 3/2-Wegeventils an unterschiedlichen Positionen im Dialysierflüssigkeitskreislauf angeordnet sein und bevorzugt auch die zwei Schaltstellungen auf verschiedene Weisen realisieren, um die Umkehr der Flussrichtung auf der Dialysierflüssigkeitsseite zu ermöglichen. In der hier beschriebenen ersten bevorzugten Ausführungsform kann das Zusatzventil stromaufwärts des Bypass-Ventils angeordnet sein, welches in dem Bypass-Pfad zur Umgehung des Dialysators angeordnet ist, so dass ein stromaufwärtiger Abschnitt des ersten Zusatz-Strömungspfads gleichzeitig auch ein Abschnitt des Bypass-Pfads ist. Das Zusatzventil ist ferner bevorzugt an den stromabwärtigen Abschnitt des ersten Zusatz-Strömungspfads angeschlossen, so dass ein Fluidfluss durch den ersten Zusatz-Strömungspfad durch entsprechendes Schalten des Zusatzventils einstellbar ist.

Das heißt, das Zusatzventil kann derart in dem Bypass-Pfad angeordnet sein, dass dieses vom Dialysierflüssigkeitszulauf abzweigt, sodass die Dialysierflüssigkeit bei geöffnetem Zustand des Zusatzventils erst dieses durchfließt, bevor es zum Bypass-Ventil gelangt. In einem aktivierten Zustand des Zusatzventils, in dem beispielsweise der Eingangsfluss von dem ersten Anschluss zu sowohl dem zweiten Anschluss als auch zu dem dritten Anschluss geleitet wird, kann die Dialysierflüssigkeit einerseits durch das Zusatzventil über den zweiten Anschluss zum Bypass-Ventil und andererseits über den dritten Anschluss zum stromabwärtigen Abschnitt des ersten Zusatz-Strömungspfads fließen, um so den Dialysierflüssigkeitszulauf mit dem Dialysierflüssigkeitsablauf zu verbinden. Falls in dieser Anordnung dann das Zulaufventil sowie das Ablaufventil geschlossen sind und gleichzeitig das Bypass-Ventil stromabwärts des Zusatzventils ebenfalls geschlossen ist, fließt die Dialysierflüssigkeit über das Zusatzventil in den stromabwärtigen Abschnitt des ersten Zusatz-Strömungspfads und anschließend durch den Dialysator in einer Flussrichtung, welche entgegensetzt der Flussrichtung im Betrieb bzw. in der Therapie verläuft, und anschließend über den zweiten Zusatz-Strömungspfad ab, welcher mit dem Rückschlagventil versehen ist. Somit sperrt das Bypass-Ventil in der vorstehend beschriebenen Anordnung den weiteren Fluss im Bypass-Pfad, sodass die Dialysierflüssigkeit in den stromabwärtigen Abschnitt des ersten Zusatz-Strömungspfads fließt.

Wenn das Zusatzventil in dem aktivieren Zustand alternativ den Eingangsfluss von dem ersten Anschluss lediglich zu dem dritten Anschluss leitet, der mit dem stromabwärtigen Abschnitt des ersten Zusatz-Strömungspfads verbunden ist, kann die Flussumkehr alternativ auch ohne Schalten/ Schließen/ Deaktivieren des Bypass-Ventils realisiert werden. Wenn das Zusatzventil in Form des 3/2-Wegeventils in dem Dialysierflüssigkeitskreislauf derart angeordnet und schaltbar ist, dass es während der Therapie den Eingangsfluss zu dem Zulaufventil leitet und während des Primens den Eingangsfluss zu dem ersten Zusatz-Strömungspfad leitet, kann gegebenenfalls auf das Bypass-Ventil ganz verzichtet werden, so dass offenbarungsgemäß der Bypass-Pfad und das Bypass-Ventil nicht zwangsläufig vorhanden sein müssen. Auch in diesem Fall weist der erste Zusatz-Strömungspfad (an seinem Ende) das Zusatzventil auf.

Gemäß dem angesprochenen bevorzugten Ausführungsbeispiel ist das Zusatzventil jedoch im geschlossenen Zustand in Richtung des Bypass-Ventils geöffnet und der Kreuzungsweg in Richtung des stromabwärtigen Abschnitts des ersten Zusatz-Strömungspfads ist geschlossen. In diesem Zustand ist zudem das Bypass-Ventil geschlossen. Im geöffneten Zustand des Zusatzventils bleibt dieses in Richtung des Bypass-Ventils geöffnet und der (Kreuzungs-)Weg in Richtung des stromabwärtigen Abschnitts des ersten Zusatz-Strömungspfads ist ebenfalls geöffnet.

In einer weiteren alternativen Ausführungsform der vorliegenden Offenbarung kann das Zusatzventil im Bypass-Pfad stromabwärts des Bypass-Ventils bzw. in Flussrichtung hinter diesem angeordnet sein und die Steuereinheit eingerichtet und vorbereitet sein, die Flussrichtung auf der Dialysierflüssigkeitsseite des Dialysators durch ein Öffnen des Zusatzventils, welches gemäß diesem alternativen Ausführungsbeispiel in dem geöffneten bzw. aktivierten Zustand den Eingangsfluss von dem ersten Anschluss zu dem dritten Anschluss zum stromabwärtigen Abschnitt des ersten Zusatz-Strömungspfads bzw. weiter entlang des ersten Zusatz-Strömungspfads leitet, ein Öffnen des Bypass-Ventils, und ein Schließen des Zulaufventils sowie des Ablaufventils umzukehren.

In dieser alternativen Ausführungsform kann das 3/2-Wegeventil als Zusatzventil stromabwärts des Bypass-Ventils angeordnet sein, welches in dem Bypass-Pfad zur Umgehung des Dialysators angeordnet ist, und gleichzeitig an dem stromabwärtigen Abschnitt des ersten Zusatz-Strömungspfad angeschlossen sein bzw. in den Zusatz-Strömungspfad integriert sein.

Das heißt, das Zusatzventil kann in dem Bypass-Pfad, der vom Dialysierflüssigkeitszulauf abzweigt, alternativ hinter dem bzw. stromabwärts dem Bypass-Ventil angeordnet sein. In dieser zweiten bevorzugten Ausführungsform fließt die Dialysierflüssigkeit demnach erst durch das geöffnete Bypass-Ventil im Bypass-Pfad und anschließend durch das Zusatzventil, dessen Kreuzungsweg zum stromabwärtigen Abschnitt des ersten Zusatz-Strömungspfads geöffnet ist, falls der Dialysator entlüftet bzw. geprimed werden soll. Dadurch, dass die Dialysierflüssigkeit über den zweiten Zusatz-Strömungspfad und dem in diesem angeordneten Rückschlagventil abfließen kann, kann der Fluss auf der Dialysierflüssigkeitsseite und somit der Fluss der Dialysierflüssigkeit durch den Dialysator umgekehrt werden. Im normalen Betrieb des Dialysators, also während der Therapie, kann die Dialysierflüssigkeit im nicht umgekehrten Fluss durch den Dialysator fließen, indem bevorzugt das Bypass-Ventil geschlossen ist, während das Zulaufventil und das Ablaufventil beide geöffnet sind. Das Zusatzventil ist in diesem Fall bevorzugt geschlossen.

Durch eine derartige Anordnung des Zusatzventils stromabwärts des Bypass-Ventils ist eine Überwachung einer Fehlstellung der Ventile einfacher durchzuführen, da die Anordnung eines (im Normalbetrieb des Dialysators also während der Therapie) geschlossenen Bypass-Ventils vor dem Zusatzventil eine größere Druckamplitude bzw. ein größerer Druckunterschied zwischen dem Dialysierflüssigkeitszulauf und dem Dialysierflüssigkeitsablauf mit sich zieht. Demnach kann eine Abweichung von dieser Druckamplitude leichter erkannt/ detektiert werden. Ferner lässt sich diese alternative Ausführungsform mechanisch leichter in einen bestehenden Ventilblock integrieren, da auf eine zusätzliche Verschlauchung als Verbindung zwischen dem Zusatzventil und dem Bypass-Ventil (aus der oben beschriebenen ersten Ausführungsform) verzichtet werden kann.

In einem weiteren bevorzugten Aspekt der Offenbarung kann der zweite Zusatz-Strömungspfad mit dem Rückschlagventil (in der Flussrichtung während des Normalbetriebs betrachtet) stromabwärts des Ablaufventils mit dem Dialysierflüssigkeitsablauf fluidisch gekoppelt sein. Mit anderen Worten ausgedrückt kann der zweite Zusatz-Strömungspfad mit dem in diesem angeordneten Rückschlagventil derart zwischen dem Dialysierflüssigkeitszulauf und dem Dialysierflüssigkeitsablauf verlaufen, dass die Dialysierflüssigkeit an dem, im umgekehrten Zustand mit umgekehrter Flussrichtung geschlossenen, Ablaufventil vorbei und dem Dialysierflüssigkeitsablauf wieder zugeführt wird.

In einer weiteren bevorzugten alternativen Ausführungsform der Offenbarung kann der zweite Zusatz-Strömungspfad mit dem Rückschlagventil stromabwärts des Zusatzventils und stromaufwärts des Bypass-Ventils mit dem Bypass-Pfad fluidisch gekoppelt sein. Anders ausgedrückt kann das Rückschlagventil an den (Schlauch-) Strang vor dem Bypass-Ventil angeschlossen sein. Das heißt, der zweite Zusatz-Strömungspfad mit dem passiven Rückschlagventil kann zwischen dem Dialysierflüssigkeitszulauf und dem Bypass-Pfad, genauer gesagt an einem Abschnitt zwischen dem Zusatzventil und dem Bypass-Ventil, angeordnet sein. Diese Anordnung ermöglicht eine einfache Verschraubung des Rückschlagventils ohne eine Verschlauchung, die in der weiter oben beschriebenen Alternative notwendig wäre.

Auch diese alternative Ausführungsform, in welchem der zweite Zusatz-Strömungspfad mit dem Rückschlagventil an den Bypass-Pfad an einer Stelle stromabwärts des Zusatzventils und stromaufwärts des Bypass-Ventils angekoppelt bzw. angebunden ist, lässt sich mechanisch leichter in einen bestehenden Ventilblock integrieren, da auf eine zusätzliche Verschlauchung als Verbindung zwischen dem Zusatzventil und dem Bypass-Ventil (aus der oben beschriebenen ersten Ausführungsform) verzichtet werden kann.

In einem weiteren bevorzugten Aspekt der Offenbarung kann im Dialysierflüssigkeitszulauf ein erster Drucksensor und im Dialysierflüssigkeitsablauf ein zweiter Drucksensor angeordnet sein, über welche ein Druckunterschied zwischen dem Dialysierflüssigkeitszulauf und dem Dialysierflüssigkeitsablauf ermittelbar ist. Bevorzugt ist ein Drucksensor in der Nähe einer Flusspumpe am Dialysierflüssigkeitsablauf, beispielsweise einer Ablaufpumpe, und der andere Drucksensor in der Nähe einer Flusspumpe am Dialysierflüssigkeitszulauf, beispielsweise einer Zulaufpumpe, angeordnet, um den Druckunterschied zwischen dem Dialysierflüssigkeitszulauf und dem Dialysierflüssigkeitsablauf überwachen zu können.

In einer weiteren vorteilhaften Ausführungsform der Offenbarung kann die Steuereinheit eingerichtet und vorbereitet sein, eine Drehzahl der Ablaufpumpe in Abhängigkeit von erfassten Werten zumindest des zweiten Drucksensors, vorzugsweise des zweiten Drucksensors und des ersten Drucksensors, zu steuern oder zu regeln. Durch eine Drehzahländerung/ Flussratenänderung an der Ablaufpumpe ist der Druck am Dialysierflüssigkeitsablauf einstellbar bzw. regelbar, wodurch aktiv ein unerwünschtes Öffnen des Rückschlagventils im zweiten Zusatz-Strömungspfad verhinderbar ist, da ein Nicht-Öffnen des Rückschlagventils während einer Therapie vom Öffnungswiderstand des Rückschlagventils sowie dem Druckunterschied zwischen dem Dialysierflüssigkeitszulauf und dem Dialysierflüssigkeitsablauf abhängig ist.

Mithilfe der Drucksensoren ist ferner eine eindeutige Überwachung der Ventilsteuerung umsetzbar. Sobald das Zusatzventil geschaltet bzw. geöffnet wird, breitet sich ein Druckstoß im Hydrauliksystem aus, da der Fluss der Dialysierflüssigkeit nun über die Zusatz-Strömungspfade umgeleitet wird und Luft aus dem Dialysator entfernt wird. Hierdurch ändert sich der Signalverlauf der Drücke im Dialysierflüssigkeitszulauf bzw. - ablauf. Genauso bewirkt auch eine Fehlstellung bzw. Fehlschaltung im Zusatzventil einen Anstieg im Drucksensorsignal auf Seiten der Zulaufpumpe, da der zulaufende Fluss nicht mehr abgeführt wird. In der Therapie wird bei einer Fehlstellung im Zusatzventil der Hauptfluss nicht mehr durch den Dialysator geleitet, wodurch anormale Drucksignale durch die Drucksensoren detektiert werden.

Die Offenbarung betrifft weiterhin ein Verfahren zum automatischen Primen eines Dialysators einer extrakorporalen Blutbehandlungsmaschine, mit einer Blutseite und einer Dialysierflüssigkeitsseite, welche voneinander durch eine semipermeable Membran getrennt sind. Das Verfahren weist dabei die Schritte: vorzugsweise Befüllen der Blutseite mit einer Flüssigkeit, insbesondere Priming-Flüssigkeit; Befüllen der Dialysierflüssigkeitsseite mit der Flüssigkeit, insbesondere Priming-Flüssigkeit; vorzugsweise Ansteuern einer Ablaufpumpe in einem Dialysierflüssigkeitsablauf über eine Steuereinheit und/oder Ansteuern einer Zulaufpumpe in einem Dialysierflüssigkeitszulauf über die Steuereinheit; und Ansteuern eines Zusatzventils in Form eines 3/2-Wegeventils in einem ersten Zusatz-Strömungspfad, über welchen der Dialysierflüssigkeitszulauf und der Dialysierflüssigkeitsablauf des Dialysierflüssigkeitskreislaufs fluidisch verbindbar sind, eines Zulaufventils im Dialysierflüssigkeitszulauf sowie eines Ablaufventils im Dialysierflüssigkeitsablauf über die Steuereinheit derart, dass eine Flussrichtung auf der Dialysierflüssigkeitsseite des Dialysators beim Primen im Vergleich zu einer Flussrichtung bei einer Therapie umkehrbar ist, um Luft, insbesondere Luftblasen, beim Primen des Dialysators aus der semipermeablen Membran zu entfernen.

In einer weiteren vorteilhaften Ausführungsform wird im Schritt der Ansteuerung der Ventile ferner ein Bypass-Ventil in einem Bypass-Pfad des Dialysierflüssigkeitskreislaufs über die Steuereinheit angesteuert.

Vorzugsweise weist der Ansteuerungsschritt der Ventile die Schritte Öffnen des Zusatzventils, Schließen oder Öffnen des Bypass-Ventils, Schließen des Zulaufventils, und Schließen des Ablaufventils auf, um die Flussrichtung auf der Dialysierflüssigkeitsseite des Dialysators umzukehren.

Die Offenbarung betrifft weiterhin ein Computerprogramm, umfassend Befehle, die bei einer Ausführung durch einen Computer diesen veranlassen, die folgenden Verfahrensschritte, bevorzugt die voranstehend beschriebenen Verfahrensschritte, auszuführen: vorzugsweise Befüllen der Blutseite mit einer Flüssigkeit, insbesondere Priming-Flüssigkeit, Befüllen der Dialysierflüssigkeitsseite mit der Flüssigkeit, insbesondere Priming-Flüssigkeit, vorzugsweise Ansteuern einer Ablaufpumpe in einem Dialysierflüssigkeitsablauf über eine Steuereinheit und/oder Ansteuern einer Zulaufpumpe in einem Dialysierflüssigkeitszulauf über die Steuereinheit; und Ansteuern eines Zusatzventils in Form eines 3/2-Wegeventils in einem ersten Zusatz-Strömungspfad, über welchen der Dialysierflüssigkeitszulauf und der Dialysierflüssigkeitsablauf des Dialysierflüssigkeitskreislaufs fluidisch verbindbar sind, eines Zulaufventils im Dialysierflüssigkeitszulauf sowie eines Ablaufventils im Dialysierflüssigkeitsablauf über die Steuereinheit, um Luft, insbesondere Luftblasen, beim Primen des Dialysators aus der semipermeablen Membran zu entfernen.

Sämtliche offenbarten Einrichtungen und Konfigurationen der offenbarungsgemäßen Vorrichtung können Verfahrensschritte des offenbarungsgemäßen Verfahrens bzw. des offenbarungsgemäßen Computerprogramms sein.

### Kurzbeschreibung der Figuren

Die Offenbarung wird nachfolgend anhand vorteilhafter Ausführungsformen und unter Bezugnahme auf die dazugehörenden Figuren näher erläutert. Es zeigen:
Fig. 1 eine schematische Teilansicht der extrakorporalen Blutbehandlungsmaschine gemäß einer ersten bevorzugten Ausführungsform der Offenbarung;
Fig. 2 eine schematische Teilansicht der extrakorporalen Blutbehandlungsmaschine gemäß einer zweiten bevorzugten Ausführungsform der Offenbarung;
Fig. 3 eine schematische Teilansicht der extrakorporalen Blutbehandlungsmaschine gemäß einer dritten bevorzugten Ausführungsform der Offenbarung; und
Fig. 4 ein Flussdiagramm eines automatischen Priming-Verfahrens gemäß einer bevorzugten Ausführungsform der vorliegenden Offenbarung.

Die Figuren sind lediglich schematischer Natur und dienen ausschließlich dem Verständnis der vorliegenden Offenbarung. Es wird darauf hingewiesen, dass die Merkmale der einzelnen Ausführungsformen untereinander ausgetauscht werden sowie in einer bestimmten Kombination auftreten können.

### Detaillierte Beschreibung bevorzugter Ausführungsformen

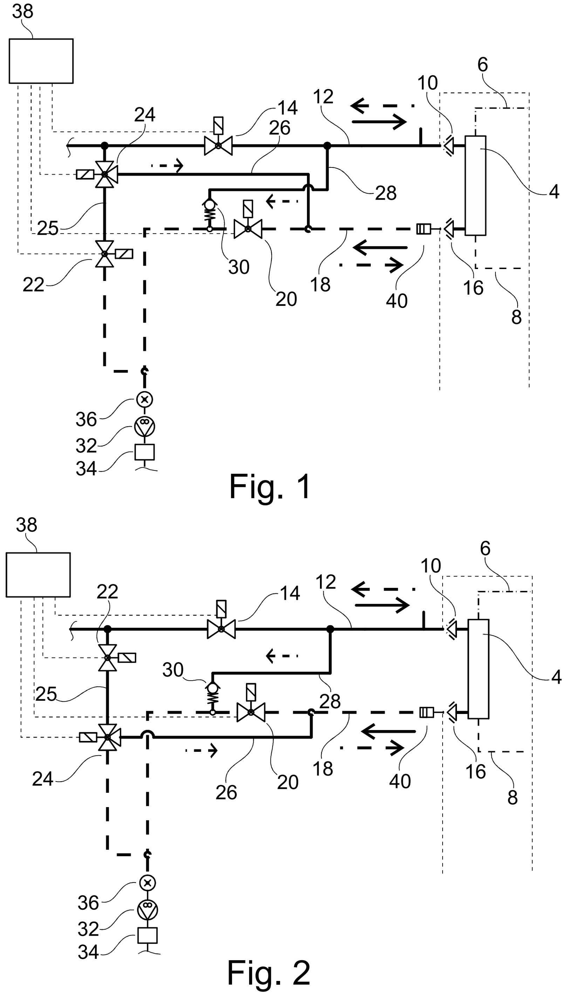

Nachfolgend wird die vorliegende Offenbarung anhand der bevorzugten Ausführungsformen mit Bezug auf die Figuren 1 bis 4 beschrieben.

Figur 1 zeigt eine schematische Teilansicht der extrakorporalen Blutbehandlungsmaschine gemäß einer ersten bevorzugten Ausführungsform der Offenbarung. Dargestellt ist ein extrakorporaler Blutkreislauf mit einem Dialysator 4, dessen Blutseite mit einem venösen Blutschlauchabschnitt 6 und einem arteriellen Blutschlauchabschnitt 8 verbunden ist. Die Dialysierflüssigkeitsseite des Dialysators 4 ist über einen ersten dialysierflüssigkeitseitigen Anschluss 10 und einen zweiten dialysierflüssigkeitsseitigen Anschluss 16 mit dem Dialysierflüssigkeitskreislauf verbunden. Vorzugsweise ist stromabwärts (betrachtet im Normalbetrieb bzw. während der Therapie der Blutbehandlungsmaschine) des zweiten dialysierflüssigkeitseitigen Anschlusses 16 ein Filter 40 vorgesehen. Der erste dialysierflüssigkeitsseitige Anschluss 10 ist dabei mit dem Dialysierflüssigkeitszulauf 12 verbunden. Der zweite dialysierflüssigkeitsseitige Anschluss 16 ist mit dem Dialysierflüssigkeitsablauf 18 fluidisch verbunden. Ferner ist ein Zulaufventil 14 in dem Dialysierflüssigkeitszulauf 12 angeordnet. In dem Dialysierflüssigkeitsablauf 18 ist ein Ablaufventil 20 angeordnet. In einem Normalbetrieb der extrakorporalen Blutbehandlungsmaschine bzw. während der Therapie fließt die Dialysierflüssigkeit auf der Dialysierflüssigkeitsseite entlang der dargestellten Pfeilrichtung durch das Zulaufventil 14 über den Dialysierflüssigkeitszulauf 12 zum Dialysator 4 und fließt anschließend über den Dialysierflüssigkeitsablauf 18 und durch das Ablaufventil 20 ab. Dabei treibt eine dem Ablaufventil 20 nachgeschaltete Ablaufpumpe 32 den Fluss der Dialysierflüssigkeit während der Therapie bzw. die Priming-Flüssigkeit auf der Dialysierflüssigkeitsseite des Dialysators 4 während des Priming-Vorgangs an. Über einen Bypass-Strömungspfad 25 mit einem Bypass-Ventil 22 ist der Dialysierflüssigkeitszulauf 12 mit dem Dialysierflüssigkeitsablauf 18 fluidisch verbindbar/trennbar und ermöglicht das Umschalten des Dialysierflüssigkeitskreislaufs in eine Bypass-Schaltung.

Die extrakorporale Blutbehandlungsmaschine weist ferner einen ersten Zusatz-Strömungspfad 26 auf. In diesem ersten Zusatz-Strömungspfad 26 ist ein Zusatzventil 24 in Form eines 3/2-Wegeventils angeordnet. Der erste Zusatz-Strömungspfad 26 kann in einem Abschnitt stromaufwärts des Zusatzventils 24 durch einen Abschnitt des Bypass-Strömungspfads 25 gebildet sein. Das Zusatzventil 24 ist derart in der Blutbehandlungsmaschine angeordnet, dass dieses stromaufwärts des Bypass-Ventils 22 angeordnet ist und mit einem stromabwärtigen Abschnitt des ersten Zusatz-Strömungspfads 26 koppelbar ist bzw. ein Fluss durch das Zusatzventil 24 entsprechend schaltbar ist. Der erste Zusatz-Strömungspfad 26 ist somit einerseits über das Zusatzventil 24 an dem Bypass-Pfad 25 und andererseits an dem Dialysierflüssigkeitsablauf 18, und zwar stromaufwärts (betrachtet im Normalbetrieb der Blutbehandlungsmaschine bzw. während der Therapie) des Ablaufventils 20 fluidisch angebunden. Des Weiteren ist der Dialysierflüssigkeitszulauf 12 und der Dialysierflüssigkeitsablauf 18 über einen zweiten Zusatz-Strömungspfad 28 fluidisch verbindbar. Dieser zweite Zusatz-Strömungspfad 28 weist ein passives Rückschlagventil 30 auf. Dabei ist der zweite Zusatz-Strömungspfad 28 einerseits stromabwärts des Zulaufventils 14 mit dem Dialysierflüssigkeitszulauf 12 und andererseits stromabwärts des Ablaufventils 20 mit dem Dialysierflüssigkeitsablauf 18 verbunden.

Mit der oben beschriebenen Anordnung der verschiedenen Strömungspfade auf Seite des Dialysierflüssigkeitskreislaufs ist die Flussrichtung durch den Dialysator bzw. der Priming-Flüssigkeit während des Primens gegenüber der Flussrichtung im Normalbetrieb, das heißt während der Therapie, umkehrbar. In den nachfolgenden Figuren ist beispielhaft die Flussrichtung während des Normalbetriebs durch durchgezogene Pfeile verdeutlicht, wohingegen die Flussrichtung während des Primens durch gestrichelte Pfeile verdeutlicht ist. Hierzu wird das Zulaufventil 14, das Ablaufventil 20 sowie das Bypass-Ventil 22 geschlossen, wohingegen das Zusatzventil 24 geöffnet wird. In diesem Zustand kann die Flüssigkeit durch das Zusatzventil 24 bis zum Bypass-Ventil 22, welches den weiteren Fluss sperrt, sowie zu dem strömabwärtigen Abschnitt des ersten Zusatz-Strömungspfads 26 hin zum Anschluss 16 des Dialysators 4 fließen. Anschließend fließt die Dialysierflüssigkeit in der vorliegenden Darstellung sowie in der Realität von unten nach oben durch den Dialysator 4 und dann durch den Dialysierflüssigkeitszulauf 12 weiter zum zweiten Zusatz-Strömungspfad 28 und, aufgrund des ausreichenden vorliegenden Druckunterschieds, durch das passive Rückschlagventil 30. Dadurch sind also das Ablaufventil 20 sowie das Zulaufventil 14 umströmbar, wodurch eine automatische Umkehrung der Fließrichtung im Dialysierflüssigkeitskreislauf ermöglicht werden kann. Im Normalbetrieb der Blutbehandlungsmaschine also während der Therapie sind das Zulaufventil 14 und das Ablaufventil 20 geöffnet, während das Bypass-Ventil 22 und das Zusatzventil 24 geschlossen sind. Ein geschlossener Zustand des Zusatzventils 24 meint hier vorzugsweise, dass ein Fluidfluss hin zum Bypass-Ventil 22 ermöglicht wird, jedoch kein Fluidfluss hin zu dem stromabwärtigen Abschnitt des ersten Zusatz-Strömungspfads 26. Im Normalbetrieb der Blutbehandlungsmaschine also während der Therapie wird bevorzugt ein Druckunterschied zwischen einem zulaufseitigen Druck und einem ablaufseitigen Druck derart eingestellt, dass der Dialysator 4 nicht über den zweiten Zusatzströmungspfad 28 umgangen wird. Beispielsweise kann ein zulaufseitiger Druck und ein ablaufseitiger Druck überwacht werden und gegebenenfalls der ablaufseitige Druck über die Pumpe 32 zumindest soweit erhöht werden, dass das Rückschlagventil 30 keinen Fluiddurchfluss ermöglicht.

Des Weiteren kommuniziert eine Steuereinheit 38 zumindest mit dem Zulaufventil 14, dem Ablaufventil 20, dem Bypass-Ventil 22 und dem Zusatzventil 24. Weiterhin sind bevorzugt Bilanzkammern 34 der Ablaufpumpe 32 nachgeschalten. Der Ablaufpumpe 32 ist in dieser Ausführungsform auch ein Drucksensor 36 vorangestellt. Vorzugsweise ist auch in dem Dialysierflüssigkeitszulauf 12 ein Drucksensor 36 vorgesehen. Auch kann in dem Dialysierflüssigkeitszulauf 12 eine Pumpe vorgesehen sein.

Figur 2 zeigt eine schematische Teilansicht der extrakorporalen Blutbehandlungsmaschine gemäß einer zweiten bevorzugten Ausführungsform der Offenbarung. Die Blutbehandlungsmaschine dieser zweiten bevorzugten Ausführungsform ist entsprechend der ersten Ausführungsform ausgebildet. Lediglich die Anordnung des Zusatzventils 24 sowie der stromabwärtige Abschnitt des ersten Zusatz-Strömungspfads 26 unterscheidet sich in dieser zweiten Ausführungsform. Hierbei ist das Zusatzventil 24 stromabwärts des Bypass-Ventils 22 im Bypass-Strömungspfad 25 angeordnet, wobei der stromaufwärtige Abschnitt des ersten Zusatz-Strömungspfads 26 ähnlich wie in der ersten Ausführungsform durch einen Abschnitt des Bypass-Strömungspfads 25 gebildet wird.

Falls eine Flussumkehr in dieser zweiten bevorzugten Ausführungsform durchgeführt werden soll, wird das Bypass-Ventil 22 im Bypass-Pfad 25 geöffnet und das Zusatzventil 24 geschlossen, so dass ein Fluidfluss lediglich zum stromabwärtigen Abschnitt des ersten Zusatz-Strömungspfad 26 ermöglicht ist. Gleichzeitig sind Zulaufventil 14 sowie Ablaufventil 20 geschlossen. Dadurch, dass die Dialysierflüssigkeit über den zweiten Zusatz-Strömungspfad 28 und dem in diesem angeordneten Rückschlagventil 30 abfließen kann, kann der Fluss auf der Dialysierflüssigkeitsseite und somit der Fluss der Flüssigkeit durch den Dialysator 4 umgekehrt werden.

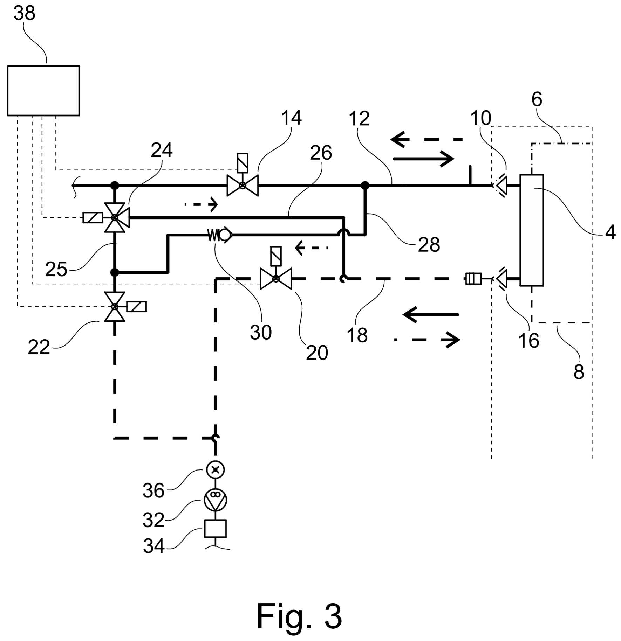

Figur 3 zeigt eine schematische Teilansicht der extrakorporalen Blutbehandlungsmaschine gemäß einer dritten bevorzugten Ausführungsform der Offenbarung. Die Blutbehandlungsmaschine dieser dritten Ausführungsform ist entsprechend der ersten Ausführungsform ausgebildet. Lediglich die Anordnung des zweiten Zusatz-Strömungspfads 28 und des passiven Rückschlagventils 30 ist unterschiedlich. Der zweite Zusatz-Strömungspfad 28 zweigt an einem Ende des Dialysierflüssigkeitszulaufs 12 ab, und zwar stromabwärts (betrachtet im Normalbetrieb der Blutbehandlungsmaschine bzw. während der Therapie) des Zulaufventils 14, und ist mit dem Bypass-Pfad 25 gekoppelt, und zwar zwischen dem Zusatzventil 24 und dem Bypass-Ventil 22. Für eine Umkehr der Flussrichtung der Dialysierflüssigkeit sind das Zulaufventil 14 und das Ablaufventil 20 geschlossen, während das Zusatzventil 24 geschlossen ist, also den Fluidfluss lediglich zu dem stromabwärtigen Abschnitt des ersten Zusatz-Strömungspfads 26 ermöglicht, und das Bypass-Ventil 22 geöffnet ist. Hierbei ist das Zusatzventil 24 somit in Richtung des Bypass-Ventils 22 geschlossen.

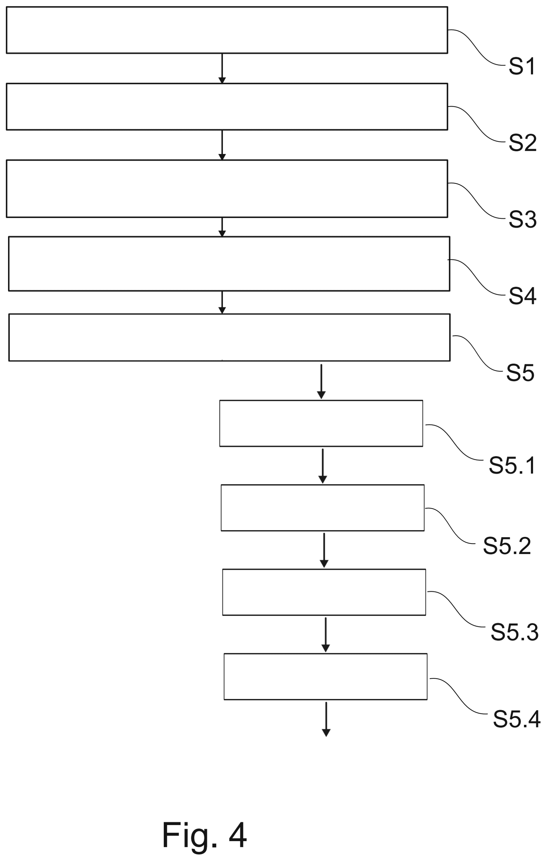

Figur 4 zeigt ein Flussdiagramm eines automatischen Priming-Verfahrens gemäß einer bevorzugten Ausführungsform der vorliegenden Offenbarung. Das Verfahren weist die Schritte Befüllen S1 der Blutseite mit einer Flüssigkeit, insbesondere Priming-Flüssigkeit, Befüllen S2 der Dialysierflüssigkeitsseite mit der Flüssigkeit, insbesondere Priming-Flüssigkeit, Ansteuern S3 einer Ablaufpumpe 32 in einem Dialysierflüssigkeitsablauf 18 über eine Steuereinheit 38, und/oder Ansteuern S4 einer Zulaufpumpe in einem Dialysierflüssigkeitszulauf 12 über die Steuereinheit 38, und Ansteuern S5 eines Zusatzventils 24 in Form eines 3/2-Wegeventils in einem ersten Zusatz-Strömungspfad 26, über welchen der Dialysierflüssigkeitszulauf 12 und der Dialysierflüssigkeitsablauf 18 des Dialysierflüssigkeitskreislaufs fluidisch verbindbar sind, eines Zulaufventils 14 im Dialysierflüssigkeitszulauf 12 sowie eines Ablaufventils 20 im Dialysierflüssigkeitsablauf 18 über die Steuereinheit 38, um eine Flussrichtung auf der Dialysierflüssigkeitsseite des Dialysators beim Primen im Vergleich zu einer Flussrichtung bei einer Therapie umzukehren, zum Entfernen von Luft, insbesondere Luftblasen, beim Primen des Dialysators 4 aus der semipermeablen Membran.

Vorzugsweise wird im Schritt der Ansteuerung der Ventile 14, 20, 24 ferner ein Bypass-Ventil 22 in einem Bypass-Pfad 25 des Dialysierflüssigkeitskreislaufs über die Steuereinheit 38 angesteuert.

Vorzugsweise weist der Schritt Ansteuern S5 der Ventile 14, 20, 24 die Schritte Ansteuern, beispielsweise Öffnen oder Schließen S5.1 des Zusatzventils 24, Ansteuern S5.2 (Schließen oder Öffnen) des Bypass-Ventils 22, Ansteuern (insbesondere Schließen) S5.3 des Zulaufventils 14, und Ansteuern (insbesondere Schließen) S5.4 des Ablaufventils 20 auf, um eine Flussrichtung auf der Dialysierflüssigkeitsseite des Dialysators 4 umzukehren.

### Bezugszeichenliste

- 4: Dialysator
- 6: venöser Blutschlauchabschnitt
- 8: arterieller Blutschlauchabschnitt
- 10: erster dialysierflüssigkeitsseitiger Anschluss
- 12: Dialysierflüssigkeitszulauf
- 14: Zulaufventil
- 16: zweiter dialysierflüssigkeitsseitiger Anschluss
- 18: Dialysierflüssigkeitsablauf
- 20: Ablaufventil
- 22: Bypass-Ventil
- 24: Zusatzventil
- 25: Bypass-Pfad
- 26: erster Zusatz-Strömungspfad
- 28: zweiter Zusatz-Strömungspfad
- 30: Rückschlagventil
- 32: Abflusspumpe
- 34: Bilanzkammern
- 36: Drucksensor
- 38: Steuereinheit
- 40: Filter

- S1: Befüllen der Blutseite
- S2: Befüllen der Dialysierflüssigkeitsseite
- S3: Ansteuern der Ablaufpumpe
- S4: Ansteuern der Zulaufpumpe
- S5: Ansteuern der Ventile
- S5.1: Ansteuern des Zusatzventils
- S5.2: Ansteuern des Bypass-Ventils
- S5.3: Schließen des Zulaufventils
- S5.4: Schließen des Ablaufventils

## Patentansprüche

1. Extrakorporale Blutbehandlungsmaschine, insbesondere Dialysemaschine, mit:
- einem Dialysator (4) mit einer Blutseite und einer Dialysierflüssigkeitsseite, welche vorzugsweise voneinander durch eine semipermeable Membran getrennt sind,
- einem Dialysierflüssigkeitskreislauf mit einem Dialysierflüssigkeitszulauf (12), welcher ein Zulaufventil (14) aufweist, einem Dialysierflüssigkeitsablauf (18), welcher ein Ablaufventil (20) aufweist, wobei der Dialysierflüssigkeitskreislauf über den Dialysierflüssigkeitszulauf (12) sowie den Dialysierflüssigkeitsablauf (18) mit der Dialysierflüssigkeitsseite des Dialysators (4) verbunden ist, **dadurch gekennzeichnet, dass**
der Dialysierflüssigkeitskreislauf einen ersten Zusatz-Strömungspfad (26) mit einem Zusatzventil (24) in Form eines 3/2-Wegeventils und einen zweiten Zusatz-Strömungspfad (28) mit einem Rückschlagventil (30) aufweist, über welche der Dialysierflüssigkeitszulauf (12) und der Dialysierflüssigkeitsablauf (18) des Dialysierflüssigkeitskreislaufs fluidisch verbindbar sind, und die Blutbehandlungsmaschine ferner eine Steuereinheit (38) aufweist, die eingerichtet ist, das Zusatzventil (24), das Zulaufventil (14) sowie das Ablaufventil (20) beim Primen derart zu steuern, dass eine Flussrichtung auf der Dialysierflüssigkeitsseite des Dialysators (4) beim Primen im Vergleich zu einer Flussrichtung bei einer Therapie umkehrbar ist.

2. Extrakorporale Blutbehandlungsmaschine nach Anspruch 1, **dadurch gekennzeichnet, dass** der Dialysierflüssigkeitskreislauf einen Bypass-Pfad (25) mit einem Bypass-Ventil (22) aufweist, über welches ein Bypass-Zustand der Blutbehandlungsmaschine einstellbar ist.

3. Extrakorporale Blutbehandlungsmaschine nach Anspruch 2, **dadurch gekennzeichnet, dass** das Zusatzventil (24) stromaufwärts des Bypass-Ventils (22) angeordnet ist und die Steuereinheit (38) eingerichtet ist und vorbereitet ist, die Flussrichtung auf der Dialysierflüssigkeitsseite des Dialysators (4) durch ein Öffnen des Zusatzventils (24), ein Schließen des Bypass-Ventils (22), und ein Schließen des Zulaufventils (14) sowie des Ablaufventils (20) umzukehren.

4. Extrakorporale Blutbehandlungsmaschine nach Anspruch 2, **dadurch gekennzeichnet, dass** das Zusatzventil (24) stromabwärts des Bypass-Ventils (22) angeordnet ist und die Steuereinheit (38) eingerichtet ist und vorbereitet ist, die Flussrichtung auf der Dialysierflüssigkeitsseite des Dialysators (4) durch ein Schließen des Zusatzventils (24), ein Öffnen des Bypass-Ventils (22), und ein Schließen des Zulaufventils (14) sowie des Ablaufventils (20) umzukehren.

5. Extrakorporale Blutbehandlungsmaschine nach einem der vorhergehenden Ansprüche, **dadurch gekennzeichnet, dass** der zweite Zusatz-Strömungspfad (28) mit dem Rückschlagventil (30) stromabwärts des Ablaufventils (20) mit dem Dialysierflüssigkeitsablauf (18) fluidisch gekoppelt ist.

6. Extrakorporale Blutbehandlungsmaschine nach Anspruch 2 oder 3, **dadurch gekennzeichnet, dass** der zweite Zusatz-Strömungspfad (28) mit dem Rückschlagventil (30) stromabwärts des Zusatzventils (24) und stromaufwärts des Bypass-Ventils (22), also zwischen dem Zusatzventil (24) und dem Bypass-Ventil (22) mit dem Bypass-Pfad (25) fluidisch gekoppelt ist.

7. Extrakorporale Blutbehandlungsmaschine nach einem der vorstehenden Ansprüche, **dadurch gekennzeichnet, dass** im Dialysierflüssigkeitszulauf (12) ein erster Drucksensor (36) und im Dialysierflüssigkeitsablauf (18) ein zweiter Drucksensor (36) angeordnet ist, über welche ein Druckunterschied zwischen dem Dialysierflüssigkeitszulauf (12) und dem Dialysierflüssigkeitsablauf (18) ermittelbar ist.

8. Extrakorporale Blutbehandlungsmaschine nach Anspruch 7, **dadurch gekennzeichnet, dass** die Steuereinheit (38) eingerichtet und vorbereitet ist, eine Drehzahl oder Flussrate einer Pumpe, vorzugsweise einer Ablaufpumpe (32) oder einer Zulaufpumpe, in Abhängigkeit von erfassten Werten des ersten Drucksensors (36) und/oder des zweiten Drucksensors (36) zu steuern oder zu regeln.

9. Verfahren zum automatischen Primen eines Dialysators (4) einer extrakorporalen Blutbehandlungsmaschine, vorzugsweise nach einem der vorhergehenden Ansprüche 1 bis 8, mit einer Blutseite und einer Dialysierflüssigkeitsseite, welche vorzugsweise voneinander durch eine semipermeable Membran getrennt sind, mit den Schritten:
- Befüllen (S2) eines Dialysierflüssigkeitskreislaufs der extrakorporalen Blutbehandlungsmaschine, insbesondere der Dialysierflüssigkeitsseite des Dialysators (4) mit einer Flüssigkeit, insbesondere Priming-Flüssigkeit; und dabei zumindest
- Ansteuern (S5) eines Zusatzventils (24) in Form eines 3/2-Wegeventils in einem ersten Zusatz-Strömungspfad (26), über welchen ein Dialysierflüssigkeitszulauf (12) und ein Dialysierflüssigkeitsablauf (18) des Dialysierflüssigkeitskreislaufs fluidisch verbindbar sind, eines Zulaufventils (14) im Dialysierflüssigkeitszulauf (12) sowie eines Ablaufventils (20) im Dialysierflüssigkeitsablauf (18) über eine Steuereinheit derart, dass eine Flussrichtung auf der Dialysierflüssigkeitsseite des Dialysators (4) beim Primen im Vergleich zu einer Flussrichtung bei einer Therapie umkehrbar ist, um Luft, insbesondere Luftblasen, beim Primen des Dialysators (19) aus der semipermeablen Membran zu entfernen.

10. Verfahren nach Anspruch 9, **dadurch gekennzeichnet, dass** im Schritt Ansteuern (S5) ferner ein Bypass-Ventil (22) in einem Bypass-Pfad (25) des Dialysierflüssigkeitskreislaufs über die Steuereinheit (38) angesteuert wird.

11. Verfahren nach Anspruch 10, **dadurch gekennzeichnet, dass** der Schritt Ansteuern (S5) die folgenden Schritte umfasst:
- Ansteuern (S5.1), insbesondere Öffnen oder Schließen, des Zusatzventils (24);
- Ansteuern (S5.2), insbesondere Öffnen oder Schließen, des Bypass-Ventils (22);
- Schließen (S5.3) des Zulaufventils (14); und
- Schließen (S5.4) des Ablaufventils (20),
um die Flussrichtung auf der Dialysierflüssigkeitsseite des Dialysators (4) beim Primen umzukehren.

12. Computerprogramm, umfassend Befehle, die bei einer Ausführung durch einen Computer diesen veranlassen, die Verfahrensschritte gemäß dem Verfahren nach einem der Ansprüche 9 bis 11 auszuführen.
